(19)

**Europäisches Patentamt**
**European Patent Office**
**Office européen des brevets**

(11) **EP 3 566 728 A1**

(12) **EUROPEAN PATENT APPLICATION**

(43) Date of publication:
**13.11.2019 Bulletin 2019/46**

(51) Int Cl.:
**A61M 1/10** *(2006.01)* **A61M 1/12** *(2006.01)*
**F04B 43/06** *(2006.01)* **F04B 43/00** *(2006.01)*

(21) Application number: **18171440.3**

(22) Date of filing: **09.05.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **ETH Zürich**
**8092 Zürich (CH)**

(72) Inventors:
• **LOOSLI, Christian**
**8057 Zürich (CH)**

• **KRESS, Gerald**
**8052 Zürich (CH)**
• **HOPF, Raoul**
**3011 Bern (CH)**
• **MAZZA, Edoardo**
**5430 Wettingen (CH)**

(74) Representative: **Bremi, Tobias Hans**
**Isler & Pedrazzini AG**
**Giesshübelstrasse 45**
**Postfach 1772**
**8027 Zürich (CH)**

(54) **CORRUGATED MEMBRANES**

(57) A corrugated circular membrane (1) is proposed, in particular for a pump, including a total artificial heart. It comprises at least one corrugated circular segment (2), adjacent to a circular rim portion (3) as well as normally at least three and not more than 15 circular segments. The thickness (t) in the at least one corrugated circular segment (2) varies by less than 20% within the at least one corrugated circular segment (2) and is at least a factor of two smaller than the maximum thickness of the circular rim portion (3), and the radial shape of the at least one corrugated circular segment (2) in the position of no strain is shaped based on a series circular segments, which can at most be semi-circles, the circle centres of which are sequentially positioned along a radial direction, and wherein the circle centre of at least one of the innermost and the outermost circular segment is at a distance from a global membrane mid plane different from the distance the circle centres of the other circular segments from said global membrane mid plane.

FIG. 1

EP 3 566 728 A1

**Description**

TECHNICAL FIELD

The present invention relates to corrugated membranes in particular for ventricular assist devices or total artificial hearts as well as to methods for designing corresponding corrugated membranes and uses of such corrugated membranes, in particular having a layer of endothelial cells on at least one side thereof.

PRIOR ART

In industrial nations approximately 1-2% of the population suffers from severe heart failure of which more than 10% are above the age of 70. A treatment option is the use of mechanical circulatory support devices as long-term therapy, also called destination therapy. However, a major problem associated with existing ventricular assist devices (VAD) is the contact of blood with artificial surfaces causing activation of blood coagulation with fatal consequences for the patient.

Pulsatile pumps like the Thoratec PVAD and IVAD (Thoratec Corp., Pleasanton, CA, USA), the Berlin Heart EXCOR (Berlin Heart 2018) or the CARMAT total artificial heart (TAH) avoid high diaphragm membrane straining by adjusting the size of the undeformed diaphragm surface to the expanded configuration, resulting in oversize. Such sac-type diaphragms exhibit strong wrinkling during operating cycles. The diaphragm of the ReinHeart TAH on the other hand, is pre-curved close to the clamping flange. However, local buckling has been observed during inflation.

SUMMARY OF THE INVENTION

The present project aims inter alia at developing a hydraulically actuated pulsatile ventricular assist device (pVAD) membrane, preferably with full hemocompatibility and allowing covering the pump membrane surface with an endothelial cell layer. These cells are irreconcilable with high strains to which they are exposed at the pump diaphragm surface during actuation. In the following, we propose a corrugated diaphragm design for a pVAD, allowing endothelial cells to survive on its surface and thereby solving the problem of hemoincompatability.

In order for endothelial cells to survive on the surface, the cyclic strain on the contacting diaphragm surface must not exceed certain limits. Based on our knowledge of cyclic strain of human vascular endothelial cells exposed in human aorta walls, the cyclic strain-range limit $\Delta\varepsilon_{lim}$, is specified to be at most 15%.

In our approach we avoid the dilemma of having either small pumping volumes due to strain constraints or strong wrinkling by using corrugated diaphragm shapes. Corrugated diaphragms as such are well known and already applied in pVAD's. However, to the author's knowledge, no studies exist on shape optimization of an endothelialized corrugated diaphragm considering strain constraints.

We keep the maximum curvature of the corrugations low by selecting corrugation shapes based on circular sections with constant curvature per section. We search for maximum pumping volumes by solving a shape optimization problem under the constraint of limited diaphragm-surface strain.

As for the function, at least one of the following targets is aimed for:

- Larger pumping volume by same amount of surface strain with corrugated shape
- Compared to sac-type membranes the inflation of corrugated shaped membranes happens without any wrinkling in the curved surfaces
- Use of (elastomer) materials with limited surface strain capacity, for instance in a pulsatile ventricular assist device covered with an endothelial cell layer.

As for the design, at least one of the following targets is aimed for:

- Corrugated shape consists of a series of circular, C1-continuous sections having alternating circular curvature signs
- Circular sections are defined by radius and position of the center points
- Essentially constant thickness of membrane, at least in the corrugated segment
- Increased thickness of the rim by using a quadratic shape function
- Shape linear scalable to any membrane radius and membrane thickness.

The investigated diaphragm shape consists of three corrugations units and has a radius of 30mm and a constant thickness of 0.5mm. The rim has a thickness of 1mm. Surface strain limitation of $\Delta\varepsilon$=15%.

According to a first aspect of the present invention, it relates to a corrugated circular (diaphragm) membrane as defined in claim 1, in particular for a pulsatile ventricular assist device, including a total artificial heart. This membrane comprises at least one corrugated circular segment, preferably adjacent to a circular rim portion.

The radial shape of the at least one corrugated circular segment in the position of no strain is shaped based on a sequential series of at least two or three circular segments, preferably at least four, at least five or at least six or seven circular segments with alternating curvature signs, which can at most be semi-circles. Further preferably the radial shape of the at least one corrugated circular segment is based on not more than 15 such circular segments, more preferably not more than 12 or 10 such circular segments.

Preferably the corrugated circular segment spanning in radial direction at least over 50% of the radius of the full circle, more preferably over at least 70% of the radius of the full circle of the circular membrane.

The corrugated circular segment may be just the form of a ring, it may however also take the form of a full disc, so the first corrugation starting at the disc center.

The thickness (measured perpendicular to a global membrane mid plane) in the at least one corrugated circular segment of the membrane varies by less than 20% within the at least one corrugated circular segment. Furthermore, this thickness (measured perpendicular to a global membrane mid plane) in the at least one corrugated circular segment of the membrane is at least a factor of two smaller than the maximum thickness of the circular rim portion (also measured perpendicular to a global membrane mid plane).

Furthermore the radial shape of the at least one corrugated circular segment in the reference configuration (position of no strain, stress-free equilibrium state of the circular membrane) is shaped based on a series of circular segments, which can at most be semi-circles. The circle centres are sequentially positioned along a radial direction, preferably in an equally spaced manner, and wherein the circle centre of at least one of the innermost and the outermost circular segment is at a distance (different from zero) from a global membrane mid plane. Preferably the distance of the circle centre of said at least one of the innermost and the outermost circular segment is different from the distance the circle centres of the other circular segments from said global membrane mid plane.

Such a corrugated shape membrane can withstand a large number of cycles without damage. Furthermore it is possible to grow a confluent endothelial cell layer, preferably a monolayer, on top of such a corrugated structure, and in spite of the deformations, the endothelial cells in the form of this monolayer remains stable on top of this corrugated structure. This can be assisted by additionally providing a surface topography, for example in the form of hexagonal wells, e.g. a two-dimensional array of regular hexagons formed by walls extending between a foot at the flat base surface and a top, and having a wall height perpendicular to the flat surface and a wall width measured parallel to the flat surface and perpendicular to the respective wall direction, and enclosing, with the inner edges of the foot of the walls, a well basal surface.

The thickness in the corrugated circular segment is preferably in the range of 0.3-0.8 mm, further preferably in the range of 0.4-0.6 mm.

On the other hand the maximum thickness of the circular rim portion is preferably in the range of 0.8-1.5 mm, preferably in the range of 0.9-1.1 mm.

Such a membrane is preferably based on or made from an elastomeric silicone material, preferably based on or made from a room temperature vulcanising silicone material, for example based on or made from a silicone material on the basis of poly alkylsiloxane, such as PDMS.

The membrane may comprise a circular flat centre portion, the radial extension of which is preferably at least 15%, preferably at least 20% or 25% of the total radial extension of the membrane.

On at least one side, typically the blood contacting side of the membrane it may, according to another preferred embodiment, comprise at least one preferably contiguous layer of endothelial cells, preferably in the form of a confluent monolayer of endothelial cells.

Furthermore the present invention relates to a method for making a membrane as given above and in the attached claims, wherein a solid negative template, preferably featuring photolithographic topographical structuring, is used for the molding of the membrane. A starting material solution and/or suspension and/or mixture, subsequently cured and/or cross-linked, preferably during a time in the range of 12-48 hours, preferably including a room temperature phase with the temperature in the range of 20-30°C and, if needed, an elevated temperature phase with a temperature in the range of 40-80°C, preferably in the range of 50-70°C, to form the membrane element which is then removed from the negative template, if need be followed by at least one of washing, sterilization, plasma surface treatment, including oxygen plasma surface treatment and gelatin coating of the topologically structured surface.

Furthermore the method may include a step in which endothelial cells are seeded onto the membrane and cultured for at least one day, preferably at least 2 days to generate a fully confluent monolayer.

According to yet another aspect of the present invention, it relates to the use of a membrane as given above or made using a method as given above as a flexible membrane in a biomedical device, preferably as a membrane in a ventricular assist device including a total artificial heart.

Furthermore the present invention relates to an optimisation based design method for designing membrane shapes which have an optimum geometry for the above purposes. Correspondingly therefore the present invention proposes a method for designing a corrugated circular membrane, in particular one as discussed above, as far as the geometry of the membrane with the exception of the circular rim is concerned, as defined in claim 10.

The objective is to improve the stroke volume V of a strain-limited circular membrane by using a corrugated shape for the membrane. The stroke volume V represents the enclosed volume between the maximum and minimum inflated membrane surface.

The corrugated shape which is used as the parameterised shape for the optimisation consists of a sequence of circular segments with alternating curvature signs. The center point coordinates of the n circular segments are $x_k, y_k, k = 1, 2, ..., n$. The origin of the axial coordinate is at the reference plane aligned with the membrane rim fixed by a clamping mechanism integrated in the pump chamber. A circular rim section as described above can be added circumferentially to the optimised corrugated shape forming the corrugated circular segment. The sequence of the radial positions $x_k$ is ordered: $x_{k+1} > x_k$. The axial positions $y_k$ are subject to said condition of alternating circular-segment curvature signs.

At its center, $x_0 = 0$, and its rim, $x_n = R$, the local membrane mid-surface is oriented parallel to the reference plane. Therefore,

$$x_0^c = 0, x_n^c = R$$

The parameters can be classified in optimization parameters and derived quantities.

The optimization parameters are: $x_k^c, y_k^c$: *circular section center points*

The derived quantities are:

*R: Membrane radius*
$d_k$: *Distance between center points*
$r_k$: *circular section radius*
$x_k^i, y_k^i$: *coordinate of intersection point between the circular sections*

Assume that other points exist for some design solution occurring at any time of the optimization process between initial design and optimized design solution.

The distances between all respective neighboring center points are calculated as by the following sequence:

$$d_K = \sqrt{(x_k^c - x_{k-1}^c)^2 + (y_k^c - y_{k-1}^c)^2}$$

Further all circular-segments radii are calculated as follows:

$$r_{k-1} = d_k - r_k$$

All circles are construed as follows:

$$(x - x_k^c)^2 + (y - y_k^c)^2 - r_k^2 = 0$$

Adjacent circular segments intersect with $C^1$ continuity on lines connecting their center points:

$$y = \frac{y_k^c - y_{k-1}^c}{x_k^c - x_{k-1}^c}(x - x_{k-1}^c) + y_{k-1}^c$$

Resolve for *x* coordinates of intersection points (choose the smaller result of the two roots):

$$x_k^i = x_k^c - \frac{r_k^2(x_k^c - x_{k-1}^c)^2}{\sqrt{r_k^2(x_k^c - x_{k-1}^c)^2((x_k^c - x_{k-1}^c)^2 + (y_k^c - y_{k-1}^c)^2)}}$$

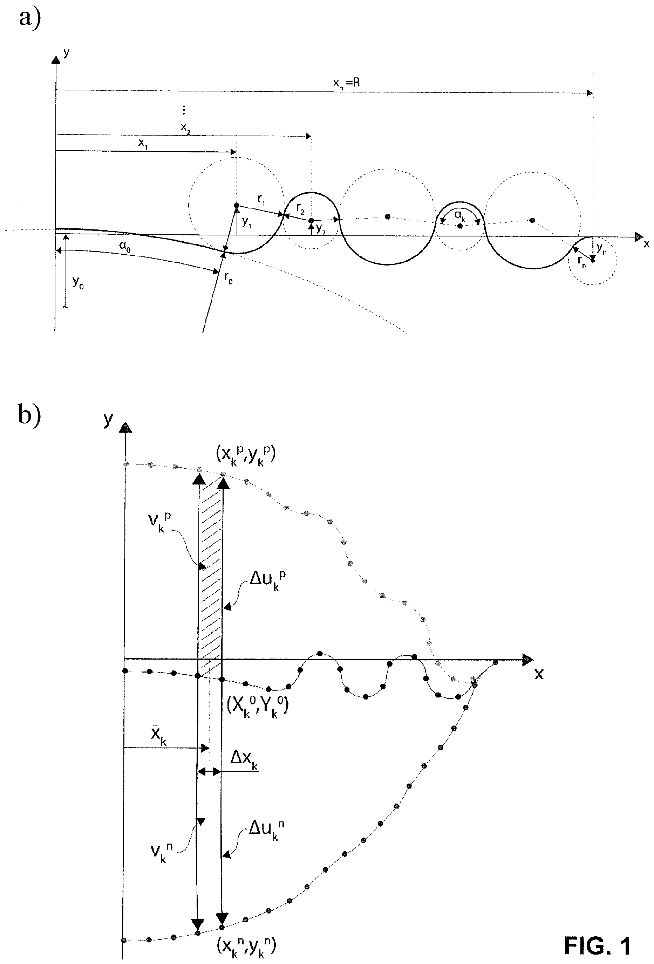

The correspondingly mathematically defined shape, which is illustrated in figure 1a), is used for the optimisation process.

In the optimisation process constraints are applied as follows:

The strain limits for the membrane are set to $\varepsilon_{max}$. To avoid manufacturing problems, the radius can be defined to be greater than a minimum radius.

To allow manufacturing of the male and female molds with standard milling tools, radii of the corrugation pattern should not be smaller than one millimeter. In order to avoid undercuts, the angle of the arch segments $\alpha_k$ should be less than or equal to 180°. To prevent contact between the inflated membrane and the chamber walls, a maximum allowed center displacement of $\Delta w_{max}$ is set, as a function of the housing geometry. The maximum allowed centre displacement can e.g. be set to the membrane radius R or just below, e.g. 2mm below the height of the housing.

For the actual optimisation process the following steps are carried out:

The membrane shape is discretized using finite elements, which partitions the profile into $n_{pts}$ nodes. In the simulation, the membrane is inflated using the nonlinear finite element method in positive and negative direction until the absolute value of the strain limit $\varepsilon_{max}$ is reached in both cases.

The resulting stroke volume is calculated with:

$$V = 2\pi \cdot \sum_{k=2}^{n_{pts}} \Delta x_k \bar{u}_k \bar{x}_k$$

where $\bar{u}_k$ is calculated with:

$$\Delta u_k = u_k^p - u_k^n = y_k^p - y_k^n$$

$$\Delta u_{k-1} = u_{k-1}^p - u_{k-1}^n = y_{k-1}^p - y_{k-1}^n$$

$$\bar{u}_k = \frac{1}{2}(\Delta u_k + \Delta u_{k-1})$$

And $\Delta x_k$, $\bar{x}_k$ are calculated with:

$$\Delta x_k = x_k^p + x_k^n - x_{k-1}^p - x_{k-1}^n$$

$$\bar{x}_k = \frac{1}{4}(x_k^p + x_k^n + x_{k-1}^p + x_{k-1}^n)$$

The optimisation is carried out by calculating the deformation of the membrane using finite element methods to an extent such that the strain limit is just reached. For a given shape this is done for the maximum positive inflation and for the maximum negative inflation and the enclosed volume between the two maximum positions is calculated.

This is illustrated in figure 1 b).

The optimisation parameters are varied until the maximum value for this enclosed volume is reached.

More generally speaking, the proposed method for the determination of an optimum corrugated circular segment, in particular for a corrugated circular membrane as defined above, it is proposed, wherein the corrugated circular segment in the un-inflated position is defined by a sequence of circular segments with alternating curvature signs, subject to the conditions that

- the origin of the axial coordinate of the corrugated circular segment is at a reference plane aligned with a rim of the corrugated circular segment;
- at the origin and at its rim the local surface of the corrugated circular segment is oriented parallel to the reference plane;
- at the intersections of adjacent circular segments the tangents to both segments are collinear;
- in the axial sequence adjacent circular segments have alternating curvature signs;

- the arc angle $\alpha_k$ of all the circular segments is less than or equal 180°;

and wherein using finite element methods this shape, fixed at its rim, is expanded in both directions until in each case a strain limit value is reached, the enclosed volume is calculated, and this enclosed volume is maximised as a function of the coordinates of the circular section centre points of the circular segments.

Furthermore the present invention relates to a corrugated circular segment obtainable or obtained using a method as detailed above.

Further embodiments of the invention are laid down in the dependent claims.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

Fig. 1    shows schematically how circular segments are used for shaping the corrugations of the corrugated circular segment, wherein in a) the shape design using a sequence of circular segments is illustrated for the uninflated state, and in b) the uninflated state is illustrated as well as the maximum inflation in the positive and in the negative direction as well as the volume calculation;

Fig. 2    shows a radial cut through a membrane illustrating the rim design;

Fig. 3    shows a scheme of pulsatile ventricular assist device including corrugated diaphragm; and

Fig. 4    shows the POM mold on the left-hand side in the vacuum press on the right-hand side.

DESCRIPTION OF PREFERRED EMBODIMENTS

Fig. 1 shows schematically how circular segments are used for shaping the corrugations. The number of circular sections n is in this case 7, and the circle centre points are sequentially positioned in a radial direction.

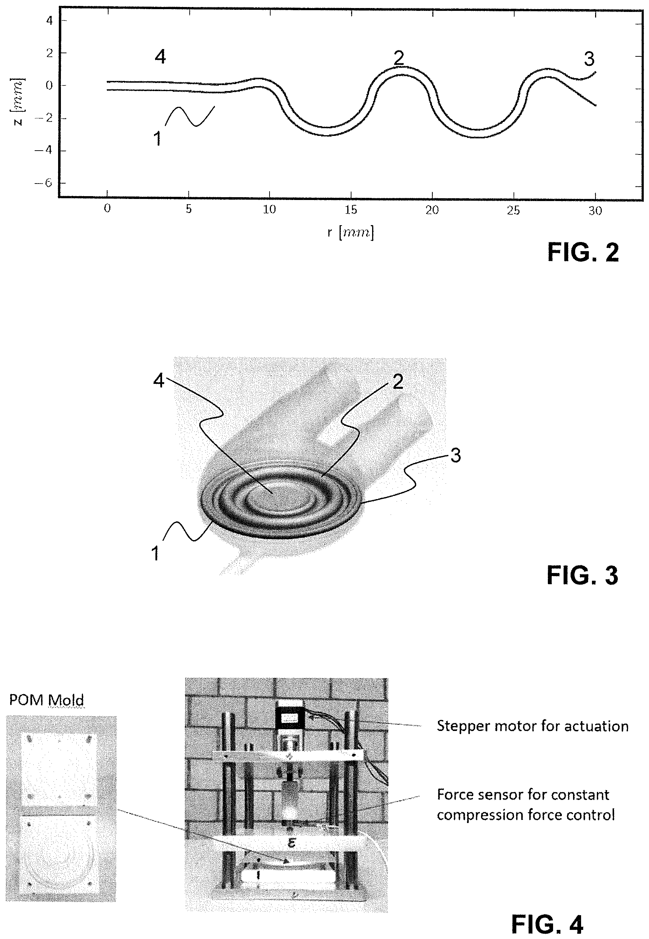

Fig. 2 shows a radial cut through a membrane illustrating the rim design. The radius of the circular segments is 30 mm and the membrane thickness in the corrugated circular segment is constant and 0.5 mm. The maximum thickness of the rim at the very terminal edge is 1 mm. The surface strain limitation is 15%.

Fig. 3 shows a scheme of pulsatile ventricular assist device including corrugated diaphragm.

***Experimental section for the fabrication of a silicone elastomer based part, for the application in a novel concept for a VAD device***

1) Corrugated Membrane

The mold for the corrugated membrane consists of two parts (a top and a bottom), between which the silicone elastomer is cured. The mold components are milled from Polyoxymethylene (POM) using a high accuracy CNC milling machine. The mold is equipped with two small outlet holes, which allow excess silicone to flux out during the molding process. Further, in all four corners of the mold, centering pins are positioned in corresponding holes. This allows the controlled closing of the molds during manufacturing. The fabrication of the elastomer membrane encompasses four main steps. The first step is preparing the silicone, followed by pouring it into the mold and then, the mold is closed inside a vacuum chamber and curing the silicone. Finally, the membrane is removed from the mold.

Procedure Description

1. Silicone preparation: Both components of the room temperature vulcanizing (RTV) silicone elastomer are weighed according to the mass ratio provided by the manufacturer (in this case 1:1). The elastomer used in this application is that Silbione® RTV 4420 by Elkem Silicones. This material cures within two hours at room temperature and has linear elastic material parameters that consist of a Young's modulus of E=455MPa and the Poisson's ratio of v=0.5 (corresponding to an incompressible material). After curing the RTV 4420 bonds weakly to the POM mold, and therefore is very easy to detach and remove from the mold without further use of any mold release agent. After both components are thoroughly mixed by hand, the resulting viscose silicone has a potting time of 30 minutes. During the first 10 minutes of this time, the silicone is de-gassed in a vacuum chamber in order to remove all the air inclusions which have been introduced during the mixing process.

2. Vacuum chamber molding: in the first step, the POM mold is thoroughly cleaned with ethanol and subsequently all residual dust particles are removed with compressed air. Then, the degassed silicone is poured onto the surface of the bottom part of the mold, such that there is more silicone in the mold than required to make the membrane. Figure 4 shows the POM mold on the left-hand side in the vacuum press on the right-hand side.

The top part is placed on top of the bottom part, in such a way that it does not touch the silicone deposited in it. Static friction on the pins prevents the mold from closing immediately, just by the pull of gravity. A custom-made machine was designed which allows the remote controlled closing of the mold inside the vacuum chamber. The machine consists of a stepper motor, which drives the spindle that translates into vertical motion of the compression plate. The force control system is implemented, such that plates remain under constant compressive force. The machine and mold are then placed inside the vacuum chamber and finally the mold is closed inside the vacuum. As soon as the mold is closed, the air inlet of the vacuum chamber is opened. Then the silicone is cured at room temperature for two hours. During this time the mold remains closed, compressed with the constant force. This process allows the fabrication high quality elastomer membranes, which are free of air inclusions.

3. When the silicone is cured, the mold can be opened without excessive use of force. Parts of the surface of the mold, can be replaced.

## LIST OF REFERENCE SIGNS

| | | | |
|---|---|---|---|
| 1 | diaphragm membrane | $r_k$ | Circular section radius |
| 2 | corrugated circular segment | t | thickness in the corrugated |
| 3 | rim portion | | circular segment |
| 4 | flat central portion | $V\ x_k^c, y_k^c$ | stroke volume |
| $d_k$ | Distance between center points | $x_k^i, y_k^i$ | Circular section centre points |
| $\varepsilon_{max}$ | Strain limit | | coordinate of intersection point between the circular sections |
| R | membrane radius | | |

## Claims

1. Corrugated circular membrane (1), in particular for a pulsatile ventricular assist device, including a total artificial heart, wherein it comprises at least one corrugated circular segment (2), adjacent to a circular rim portion (3), wherein the thickness (t) in the at least one corrugated circular segment (2) varies by less than 20% within the at least one corrugated circular segment (2) and is at least a factor of two smaller than the maximum thickness of the circular rim portion (3), and wherein the radial shape of the at least one corrugated circular segment (2) in the position of no strain is shaped based on a sequential series of at least three circular segments with alternating curvature signs, which can at most be semi-circles, wherein the circle centre of at least one of the innermost and the outermost circular segment is at a distance from a global membrane mid plane.

2. Membrane according to claim 1, wherein the circle centres of said series of circular segments are sequentially positioned and preferably equally spaced along a radial direction, and/or wherein the radial shape of the at least one corrugated circular segment (2) in the position of no strain is shaped based on a sequential series of at least four, at least five or at least six or seven circular segments with alternating curvature signs, which can at most be semi-circles, and/or preferably based on not more than 15 such circular segments, more preferably not more than 12 or 10 such circular segments; and/or wherein the distance of the circle centre of at least one of the innermost and the outermost circular segment is different from the distance the circle centres of the other circular segments from said global membrane mid plane.

3. Membrane according to any of the preceding claims, wherein the thickness (t) in the corrugated circular segment (2) is in the range of 0.3-0.8 mm, preferably in the range of 0.4-0.6 mm, and/or wherein the maximum thickness of the circular rim portion (3) is in the range of 0.8-1.5 mm, preferably in the range of 0.9-1.1 mm.

4. Membrane according to any of the preceding claims, wherein it is based on or made from an elastomeric silicone material, preferably based on or made from a room temperature vulcanising silicone material, further preferably based on or made from a silicone material on the basis of poly siloxane, in particular polyalkylsiloxane.

5. Membrane according to any of the preceding claims, wherein it comprises a circular flat centre portion (4), the radial extension of which is at least 15%, preferably at least 20% or 25% of the total radial extension of the membrane.

6. Membrane according to any of the preceding claims, wherein on at least one side thereof it comprises at least one contiguous layer of endothelial cells, preferably in the form of a confluent monolayer of endothelial cells.

7. Method for making a membrane according to any of the preceding claims, wherein a solid negative template, preferably using CNC milling, is used for molding, with a starting material solution and/or suspension and/or mixture, subsequently cured and/or cross-linked, preferably during a time in the range of 12-48 hours, preferably including a room temperature phase with the temperature in the range of 20-30°C and an elevated temperature phase with a temperature in the range of 40-80°C, preferably in the range of 50-70°C, to form the membrane element which is then removed from the negative template, if need be followed by at least one of washing, sterilization, plasma surface treatment, including oxygen plasma surface treatment and gelatin coating of the topographically structured surface.

8. Method according to claim 7, wherein endothelial cells are seeded onto the membrane and cultured for at least one day, preferably at least 2 days to generate a fully confluent monolayer.

9. Use of a membrane according to any of the preceding claims as a flexible membrane in a biomedical device, preferably as a membrane in a pulsatile ventricular assist device including a total artificial heart.

10. Method for the determination of an optimum corrugated circular segment (2), in particular for a corrugated circular membrane (1) as defined in any of the preceding claims, wherein
the corrugated circular segment (2) in the un-inflated position is defined by a sequence of circular segments with alternating curvature signs, subject to the conditions that

the origin of the axial coordinate of the corrugated circular segment (2) is at a reference plane aligned with a rim of the corrugated circular segment;
at the origin and at its rim the local surface of the corrugated circular segment (2) is oriented parallel to the reference plane;
at the intersections of adjacent circular segments the tangents to both segments are collinear;
in the axial sequence adjacent circular segments have alternating curvature signs;
the arc angle of all the circular segments is less than or equal 180°;

and wherein using finite element methods this shape, fixed at its rim, is expanded in both directions until in each case a strain limit value ($\varepsilon_{max}$) is reached, the enclosed volume ($\Delta V$) is calculated, and this enclosed volume ($\Delta V$) is maximised as a function of the coordinates $(x_k^c, y_k^c)$ of the circular section centre points of the circular segments.

11. Method according to claim 10, wherein the number of circular segments is in the range of 3-10, preferably 5-8.

12. Method according to any of claims 10 or 11, wherein for the definition of the un-inflated corrugated circular segment (2), at its center, $x_0 = 0$, and its rim, $x_n = R$, the local membrane mid-surface is oriented parallel to the reference plane as follows:

$$x_0^c = 0, x_n^c = R$$

the distances between all respective neighboring center points are calculated as follows:

$$d_K = \sqrt{(x_k^c - x_{k-1}^c)^2 + (y_k^c - y_{k-1}^c)^2}$$

all circular-segments radii are calculated as follows:

$$r_{k-1} = d_k - r_k$$

all circles are construed as follows:

$$(x - x_k^c)^2 + (y - y_k^c)^2 - r_k^2 = 0$$

adjacent circular segments are defined to intersect with $C^1$ continuity on lines connecting their center points:

$$y = \frac{y_k^c - y_{k-1}^c}{x_k^c - x_{k-1}^c}(x - x_{k-1}^c) + y_{k-1}^c$$

which is resolved for $x$ coordinates of intersection points, choosing the smaller result of the two:

$$x_k^i = x_k^c - \frac{r_k^2\left(x_k^c - x_{k-1}^c\right)^2}{\sqrt{r_k^2\left(x_k^c - x_{k-1}^c\right)^2\left(\left(x_k^c - x_{k-1}^c\right)^2 + \left(y_k^c - y_{k-1}^c\right)^2\right)}}.$$

13. Method according to any of claims 10-12, wherein for the optimisation the strain limits for the corrugated circular segment (2) are set ($\varepsilon_{max}$), optionally the radii of all circular segments are restricted to be greater than a minimum radius,
the angle of the arc segments ($\alpha_k$) of all circular segments is restricted to be less than or equal to 180°, optionally a maximum allowed center displacement (of $\Delta w_{max}$) is set.

14. Method according to any of claims 10-13, wherein for the optimisation the membrane shape is discretized using finite elements, which partitions the profile into $n_{pts}$ nodes;
for the optimisation the corrugated circular segment (2) is inflated using the nonlinear finite element method in positive and negative direction until the absolute value of the strain limit ($\varepsilon_{max}$) is reached in both cases;
the resulting stroke volume is calculated with:

$$V = 2\pi \cdot \sum_{k=2}^{n_{pts}} \Delta x_k \bar{u}_k \bar{x}_k$$

where $\bar{u}_k$ is calculated with:

$$\Delta u_k = u_k^p - u_k^n = y_k^p - y_k^n$$

$$\Delta u_{k-1} = u_{k-1}^p - u_{k-1}^n = y_{k-1}^p - y_{k-1}^n$$

$$\bar{u}_k = \frac{1}{2}(\Delta u_k + \Delta u_{k-1})$$

and $\Delta x_k$, $\bar{x}_k$ are calculated with:

$$\Delta x_k = x_k^p + x_k^n - x_{k-1}^p - x_{k-1}^n$$

$$\bar{x}_k = \frac{1}{4}(x_k^p + x_k^n + x_{k-1}^p + x_{k-1}^n).$$

**15.** Corrugated circular segment (2) obtainable or obtained using a method according to any of the preceding claims 10-14.

a)

b)

FIG. 1

FIG. 2

FIG. 3

POM Mold

Stepper motor for actuation

Force sensor for constant compression force control

FIG. 4

Europäisches
Patentamt

European
Patent Office

Office européen
des brevets

**EUROPEAN SEARCH REPORT**

Application Number

EP 18 17 1440

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/020588 A1 (SHIMIZU TOSHIHARU [JP] ET AL) 27 January 2011 (2011-01-27) | 1,2,9-14 | INV. A61M1/10 |
| A | * paragraphs [0091] - [0111]; figures 8-10 * | 5 | A61M1/12 F04B43/06 F04B43/00 |
| | ----- | | |
| X | US 2004/001766 A1 (MAIANTI EDGARDO COSTA [IT] ET AL) 1 January 2004 (2004-01-01) | 1,2 | |
| Y | * membrane 16 is corrugated; | 3,4 | |
| A | paragraph [0028]; figure 7 * | 6 | |
| | ----- | | |
| Y | US 2008/202591 A1 (GRANT KEVIN L [US] ET AL) 28 August 2008 (2008-08-28) | 3,4 | |
| A | * thickened rim; rings; high elongation silicone; thickness ranges; paragraphs [0202], [0204], [0208]; figures 5A-D * | 5 | |
| | ----- | | |
| A | US 2006/052659 A1 (TOPAZ STEPHEN R [US] ET AL) 9 March 2006 (2006-03-09) * the whole document * | 1-6 | |
| | ----- | | |

TECHNICAL FIELDS
SEARCHED      (IPC)

A61M
F04B

~~The present search report has been drawn up for all claims~~

| Place of search | Date of completion of the search | Examiner |
|---|---|---|
| The Hague | 19 October 2018 | Van Veen, Jennifer |

EPO FORM 1503 03.82 (P04C01)

Europäisches
Patentamt

European
Patent Office

Office européen
des brevets

Application Number

EP 18 17 1440

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-6, 9-14

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 18 17 1440

The Search Division considers that the present European patent application does not comply with the requirements of unity of invention and relates to several inventions or groups of inventions, namely:

1. claims: 1-6, 9-14

    Corrugated circular membrane, wherein it comprises at least one corrugated circular segment, adjacent to a circular rim portion, wherein the thickness in the at least one corrugated circular segment varies by less than 20% within the at least one corrugated circular segment and is at least a factor of two smaller than the maximum thickness of the circular rim portion, and wherein the radial shape of the at least one corrugated circular segment in the position of no strain is shaped based on a sequential series of at least three circular segments with alternating curvature signs, which can at most be semi-circles, wherein the circle centre of at least one of the innermost and the outermost circular segment is at a distance from a global membrane mid plane (cf. claim 1); wherein the circle centres of said series of circular segments are sequentially positioned (cf. claim 2); and wherein the thickness in the corrugated circular segment is in the range of 0.3-0.8 mm (cf. claim 3).
    ---

2. claims: 7, 8

    Method for making a membrane, wherein a solid negative template is used for molding, with a starting material solution and/or suspension and/or mixture, subsequently cured and/or cross-linked (cf. claim 7).
    ---

3. claim: 15

    Corrugated circular segment obtainable or obtained using a method including at least the step of calculating an enclosed volume (AV) which is maximised as a function of the coordinates (x?, yk) of the circular section centre points of the circular segments (cf. claim 15)
    ---

**ANNEX TO THE EUROPEAN SEARCH REPORT
ON EUROPEAN PATENT APPLICATION NO.**

EP 18 17 1440

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2018

| Patent document cited in search report | Publication date | Patent family member(s) | | Publication date |
|---|---|---|---|---|
| US 2011020588 A1 | 27-01-2011 | EP | 2267312 A1 | 29-12-2010 |
| | | JP | 5417317 B2 | 12-02-2014 |
| | | JP | WO2009113666 A1 | 21-07-2011 |
| | | US | 2011020588 A1 | 27-01-2011 |
| | | WO | 2009113666 A1 | 17-09-2009 |
| US 2004001766 A1 | 01-01-2004 | EP | 1362604 A1 | 19-11-2003 |
| | | IT | MI20021028 A1 | 14-11-2003 |
| | | US | 2004001766 A1 | 01-01-2004 |
| US 2008202591 A1 | 28-08-2008 | AU | 2008221370 A1 | 04-09-2008 |
| | | AU | 2008221455 A1 | 04-09-2008 |
| | | AU | 2008231167 A1 | 02-10-2008 |
| | | AU | 2014202773 A1 | 19-06-2014 |
| | | AU | 2017200864 A1 | 16-03-2017 |
| | | BR | PI0808038 A2 | 17-06-2014 |
| | | BR | PI0808040 A2 | 17-06-2014 |
| | | CA | 2681914 A1 | 04-09-2008 |
| | | CA | 2681916 A1 | 04-09-2008 |
| | | CA | 2682073 A1 | 02-10-2008 |
| | | CA | 2937204 A1 | 04-09-2008 |
| | | CN | 101678159 A | 24-03-2010 |
| | | CN | 101801432 A | 11-08-2010 |
| | | CN | 103623472 A | 12-03-2014 |
| | | CN | 104174077 A | 03-12-2014 |
| | | EP | 2131886 A1 | 16-12-2009 |
| | | EP | 2131887 A2 | 16-12-2009 |
| | | EP | 2131890 A1 | 16-12-2009 |
| | | EP | 2131893 A1 | 16-12-2009 |
| | | EP | 2735324 A1 | 28-05-2014 |
| | | EP | 3189863 A1 | 12-07-2017 |
| | | EP | 3195889 A1 | 26-07-2017 |
| | | JP | 5174044 B2 | 03-04-2013 |
| | | JP | 5291003 B2 | 18-09-2013 |
| | | JP | 5319558 B2 | 16-10-2013 |
| | | JP | 5643803 B2 | 17-12-2014 |
| | | JP | 5695129 B2 | 01-04-2015 |
| | | JP | 6001614 B2 | 05-10-2016 |
| | | JP | 6010151 B2 | 19-10-2016 |
| | | JP | 6093083 B2 | 08-03-2017 |
| | | JP | 6096400 B2 | 15-03-2017 |
| | | JP | 6208111 B2 | 04-10-2017 |
| | | JP | 2010519006 A | 03-06-2010 |
| | | JP | 2010519007 A | 03-06-2010 |
| | | JP | 2010519011 A | 03-06-2010 |
| | | JP | 2010519463 A | 03-06-2010 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 18 17 1440

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2018

| Patent document cited in search report | Publication date | Patent family member(s) | Publication date |
|---|---|---|---|
| | | JP  2013063325 A | 11-04-2013 |
| | | JP  2013176633 A | 09-09-2013 |
| | | JP  2013221524 A | 28-10-2013 |
| | | JP  2015033638 A | 19-02-2015 |
| | | JP  2015039639 A | 02-03-2015 |
| | | JP  2015083274 A | 30-04-2015 |
| | | JP  2016195793 A | 24-11-2016 |
| | | JP  2017023766 A | 02-02-2017 |
| | | JP  2017124186 A | 20-07-2017 |
| | | JP  2017227218 A | 28-12-2017 |
| | | KR  20090127144 A | 09-12-2009 |
| | | KR  20100014608 A | 10-02-2010 |
| | | KR  20150001853 A | 06-01-2015 |
| | | KR  20150058389 A | 28-05-2015 |
| | | KR  20160040304 A | 12-04-2016 |
| | | KR  20170029018 A | 14-03-2017 |
| | | KR  20170096230 A | 23-08-2017 |
| | | KR  20180027652 A | 14-03-2018 |
| | | KR  20180029281 A | 20-03-2018 |
| | | KR  20180085073 A | 25-07-2018 |
| | | KR  20180086287 A | 30-07-2018 |
| | | MX     336999 B | 08-02-2016 |
| | | US  2008202591 A1 | 28-08-2008 |
| | | US  2008208103 A1 | 28-08-2008 |
| | | US  2008208111 A1 | 28-08-2008 |
| | | US  2008253911 A1 | 16-10-2008 |
| | | US  2008253912 A1 | 16-10-2008 |
| | | US  2009004033 A1 | 01-01-2009 |
| | | US  2011299358 A1 | 08-12-2011 |
| | | US  2012207627 A1 | 16-08-2012 |
| | | US  2013074959 A1 | 28-03-2013 |
| | | US  2013177457 A1 | 11-07-2013 |
| | | US  2014153356 A1 | 05-06-2014 |
| | | US  2015125319 A1 | 07-05-2015 |
| | | US  2015196698 A1 | 16-07-2015 |
| | | US  2016175505 A1 | 23-06-2016 |
| | | US  2017130705 A1 | 11-05-2017 |
| | | US  2018038357 A1 | 08-02-2018 |
| | | US  2018372084 A1 | 27-12-2018 |
| | | WO  2008106440 A1 | 04-09-2008 |
| | | WO  2008106538 A2 | 04-09-2008 |
| US 2006052659    A1 | 09-03-2006 | NONE | |

EPO FORM P0459

For more details about this annex : see Official Journal of the European Patent Office, No. 12/82

page 2 of 2